(19) Europäisches Patentamt
European Patent Office
Office européen des brevets

(11) Veröffentlichungsnummer: **0 072 926**
**B1**

# (12) EUROPÄISCHE PATENTSCHRIFT

(45) Veröffentlichungstag der Patentschrift: **24.09.86**

(51) Int. Cl.⁴: **C 07 D 471/04**, A 61 K 31/44 // (C07D471/04, 235:00, 221:00)

(21) Anmeldenummer: **82106638.8**

(22) Anmeldetag: **23.07.82**

## (54) 2-Aryl-imidazopyridine.

(30) Priorität: **19.08.81 DE 3132754**
**01.10.81 DE 3139064**

(43) Veröffentlichungstag der Anmeldung: **02.03.83 Patentblatt 83/9**

(45) Bekanntmachung des Hinweises auf die Patenterteilung: **24.09.86 Patentblatt 86/39**

(84) Benannte Vertragsstaaten: **AT BE CH DE FR GB IT LI NL SE**

(56) Entgegenhaltungen:
**EP-A-0 022 495**
**DE-A-2 305 339**

**CHEMICAL ABSTRACTS, Band 88, Nr. 3, 16. Januar 1978, Seite 637, Nr. 22904s, Columbus, Ohio, USA**
**JOURNAL OF HETEROCYCLIC CHEMISTRY, Band 17, Dezember 1980, Seiten 1757-1760, Tampa, Florida, USA R.W. MIDDLETON et al.: "Synthesis of imidazo(4,5-b)- and (4,5-c)pyridines"**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Jonas, Rochus, Dr., Telemannweg 24, D-6100 Darmstadt (DE)**
Erfinder: **Minck, Klaus, Dr., Büchestrasse 8, D-6105 Ober- Ramstadt (DE)**
Erfinder: **Enenkel, Hans- Joachim, Dr., Wingertsbergstrasse 5, D-6100 Darmstadt (DE)**
Erfinder: **Schliep, Hans- Jochen, Dr., Weingartenstrasse 16, D-6109 Traisa (DE)**

**EP 0 072 926 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue 2-Aryl-imidazopyridine der allgemeinen Formel

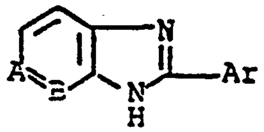

I

worin

-A=B- (a) -CH = N- oder (b) -N=CH-,

Ar einen Phenylrest, der im Fall (a) durch eine oder zwei Alkinyloxy-, Cyanmethoxy- und/oder Alkyloxycarbonylmethoxygruppen substituiert ist und durch eine oder zwei zusätzliche Hydroxy-, Alkyloxy-, Alkenyloxy- und/oder Alkinyloxygruppen substituiert sein kann, oder der im Fall (b) durch eine bis drei Hydroxy-, Mercapto- und/oder -Z-R-Gruppen substituiert ist,

Z -O-, -S- oder -SO- und

R Alkyl, Alkenyl, Alxinyl, Hydroxyalkyl, Cyanmethyl oder Alkyloxycarbonylmethyl bedeuten,

wobei die Alkyl-, Alkenyl-, Alkinyl- und Hydroxyalkylgruppen jeweils bis zu 5 C-Atome besitzen, worin jedoch im Fall (b) der Phenylrest nur dann durch Hydroxy- oder Methoxygruppen substituiert ist, wenn er gleichzeitig noch mindestens einen anderen jeweils davon verschiedenen Substituenten trägt,

sowie ihre physiologisch unbedenklichen Salze.

Bei der Synthese dieser neuen Verbindungen entsteht nur eine Form, für die die Struktur der 3H-Imidazopyridine (I) angenommen wird, im einzelnen die der 3H-Imidazo-(4,5-b)pyridine (Ia; vgl. "The Ring-Index", 2nd Edition, American Chemical Society, 1980, Nr. 1193) bzw. die der 3H-Imidazo(4,5-c)pyridine (Ib; vgl. "The Ring Index", 1.c., Nr. 1195).

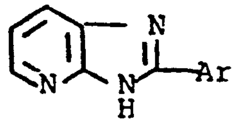

Ia

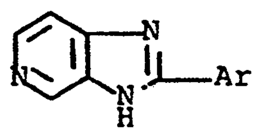

Ib

Die Struktur der tautomeren 1H-Imidazopyridine I', im einzelnen die der 1H-Imidazo(4,5-b)pyridine (Ia'; "The Ring Index", 1.c., Nr. 1192) bzw. die der 1H-Imidazo(4,5-c)pyridine (Ib'; "The Ring-Index", 1.c. Nr. 1194)

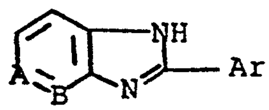

I'

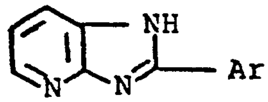

Ia'

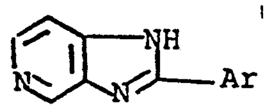

Ib'

ist jedoch ebenfalls möglich. Die Verbindungen werden daher vor- und nachstehend als "Imidazopyridine" bzw. "Imidazo(4,5-b)pyridine" bzw. "Imidazo(4,5-c)pyridine" oder "Verbindungen der Formel I" bzw. "Verbindungen der Formel Ia" bzw. Verbindungen der Formel Ib" bezeichnet; diese Bezeichnungen sollen sowohl die 3H- I bzw. Ia bzw. Ib als auch die 1H-Tautomeren I' bzw. Ia' bzw. Ib' einschließen. Ähnliche Verbindungen sind aus der DE-OS 2 305 339 bekannt. Auch in der SU-PS 566 842 sind ähnliche Verbindungen beschrieben, z.B. solche, die der Formel Ib entsprechen, worin jedoch an Stelle von Ar eine Phenyl- oder eine p-Methoxyphenylgruppe steht. Weiterhin ist 2-o-Hydroxyphenylimidazo(4,5-c)pyridin aus J. Het. Chem., Band 17, Seiten 1757 - 1760 (1980) bekannt.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I und ihrer physiologisch unbedenklichen Salze gelöst.

Es wurde gefunden, daß diese Verbindungen bei guter Verträglichkeit wertvolle pharmakologische Eigenschaften besitzen. Insbesondere zeigen sie eine Wirkung auf den Blutdruck, die Herzkraft (positiv inotrope Wirksamkeit) und eine Ulcus-Wirkung. Die Blutdruck- und Herzwirkung kann z. B. an narkotisierten oder wachen Hunden, Katzen, Affen oder Minischweinen, die positiv inotrope Wirkung auch an isolierten Herzpräparationen (z. B. Vorhof, Papillarmuskel oder perfundiertes Ganzherz) von Ratte, Meerschweinchen oder Katze ermittelt werden, z. B. nach Methoden, wie sie in Arzneimittelforschung, Band 31 (I) Nr. 1a (1951), Seiten 141 bis 170, beschrieben sind.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind Verbindungen der Formel und ihre physiologisch unbedenklichen Salze.

In den Verbindungen der Formel I kann die Phenylgruppe einfach (in o-, m- oder p-Stellung), zweifach (in 2,3-, 2,4-, 2,5-, 2,6-, 3,4- oder 3,5-Stellung), dreifach (in 2,3,4-, 2,3,5-, 2,3,8- oder 3,4,5-Stellung) oder (im Fall (a)) auch vierfach (in 2,3,4,5-, 2,3,4,8-oder 2,3,5,6-Stellung) substituiert sein. Bevorzugt sind als Rest Ar 2,4-disubstituierte, ferner o- oder p-monosubstituierte Phenylgruppen.

Alkyl ist vorzugsweise unverzweigt, hat vorzugsweise 1 - 4, insbesondere 1 - 3 C-Atome und steht bevorzugt für Methyl, ferner für Ethyl, Propyl, Isopropyl, Butyl, Isobutyl, sek.-Butyl, tert.-Butyl, Pentyl oder Isopentyl.

Alkenyl ist vorzugsweise unverzweigt, hat insbesondere 2 - 4, bevorzugt 3 C-Atome und steht bevorzugt für Allyl, ferner für Vinyl, 1-Propen-1-yl, Butenyl wie 1-Buten-1-yl, 2-Buten-1-yl, 3-Buten-1-yl, Pentenyl wie 1-Penten-1-yl, 2-Penten-1-yl, 3-Penten-1-yl. Die Alkenylgruppen können aber auch verzweigt sein; Beispiele dafür sind 1-Methyl-2-propen-1-yl, 1-Methyl-2-buten-1-yl, 2-Methyl-3-buten-1-yl, 1,1-Dimethyl-2-propen-1-yl.

Bevorzugte Alkinylgruppen sind unverzweigt und besitzen insbesondere 3, aber auch 2, 4 oder 5 C-Atome wie Propargyl (= 2-Propin-1-yl), ferner Ethinyl, 1-Propin-1-yl, Butinyl wie 1-Butin-1-yl, 2-Butin-1-yl, 3-Butin-1-yl, Pentinyl wie 1-Pentin-1-yl, 2-Pentin-1-yl, 3-Pentin-1-yl. Die Alkinylgruppen können aber auch verzweigt sein; Beispiele dafür sind 1-Methyl-2-propin-1-yl, 1-Methyl-2-butin-1-yl, 2-Methyl-3-butin-1-yl, 1,1-Dimethyl-2-propin-1-yl.

Hydroxyalkyl ist vorzugsweise unverzweigt, hat insbesondere 2 - 4, bevorzugt 2 oder 3 C-Atome und steht bevorzugt für 2-Hydroxyethyl oder 3-Hydroxypropyl, ferner auch z. B. für Hydroxymethyl, 1-Hydroxyethyl, 1- oder 2-Hydroxypropyl, 1- oder 2-Hydroxy-1-methyl-ethyl, 1-, 2-, 3- oder 4-Hydroxkbutyl, 1-, 2-, 3-, 4- oder 5-Hydroxypentyl.

Bevorzugte Alkyloxycarbonylmethylgruppen haben 1 - 4, insbesondere 1 - 3 C-Atome im Alkoxyrest wie Methoxyund Ethoxycarbonylmethyl, ferner Propoxy-, Isopropoxy-, Butoxy-, Isobutoxy-, sek.-Butoxy-, tert.-Butoxy-, Pentoxy- und Isopentoxycarbonylmethyl.

In Verbindungen der Formel Ib sind z.B. folgende Substituenten am Phenylrest besonders bevorzugt: Hydroxy (nur wenn mindestens ein anderer von Hydroxy verschiedener Substituent am Phenylrest vorhanden ist), Methoxy (nur wenn mindestens ein anderer von Methoxy verschiedener Substituent am Phenylrest vorhanden ist), Ethoxy, Propoxy, Isopropoxy, Butoxy, Vinyloxy, Allyloxy, Propargyloxy, 2-Hydroxyethoxy, Cyanmethoxy, Methoxycarbonylmethoxy, Ethoxycarbonylmethoxy, Methylthio, Ethylthio, Propylthio, Isopropylthio, Butylthio, Vinylthio, Allylthio, Propargylthio, 2-Hydroxyethylthio, Cyanmethylthio, Methoxycarbonylmethylthio, Ethoxycarbonylmethylthio, Methylsulfinyl, Ethylsulfinyl, Propylsulfinyl, Isopropylsulfinyl, Butylsulfinyl, Vinylsulfinyl, Allylsulfinyl, Propargylsulfinyl, 2-Hydroxyethylsulfinyl, Cyanmethylsulfinyl, Methoxycarbonylmethylsulfinyl, Ethoxycarbonylmethylsulfinyl.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ic bis Iq ausgedrückt werden, die der Formel I entsprechen und worin der Rest Ar einen Phenylrest bedeutet, der wie folgt substituiert ist:

(a) im Fall -A=B- = -CH=N-:

in Ic: durch eine Alkinyloxy-, Cyanmethoxy- oder Alkyloxycarbonylmethoxygruppe und gegebenenfalls durch eine oder zwei zusätzliche Hydroxy-, Alkyloxy-, Alkenyloxy- und/oder Alkinyloxygruppen, wobei die Alkyl-, Alkenyl- und Alkinylgruppen jeweils bis zu 4 C-Atome besitzen;

in Id: durch eine Alkinyloxy-, Cyanmethoxyoder Alkyloxycarbonylmethoxygruppe und gegebenenfalls durch eine oder zwei zusätzliche Hydroxy-, Alkyloxy-, Alkenyloxy- und/oder Alkinyloxygruppen, wobei die Alkyl-, Alkenyl- und Alkinylgruppen jeweils bis zu 3 C-Atome besitzen;

in Ie: durch eine Propargyloxy-, Cyanmethoxyoder Alkyloxycarbonylmethoxygruppe und gegebenenfalls durch eine oder zwei zusätzliche Alkyloxygruppen oder durch eine zusätzliche Allyloxy- oder Propargyloxygruppe,wobei die Alkyloxygruppen jeweils 1 - 3 C-Atome besitzen;

in If: durch eine Propargyloxygruppe und gegebenenfalls zusätzlich durch eine oder zwei Methoxygruppen;

in Ig: durch eine Cyanmethoxygruppe und gegebenenfalls zusätzlich durch eine oder zwei Methoxygruppen;

in Ih: durch eine Methoxycarbonylmethoxy- oder Ethoxycarbonylmethoxygruppe und gegebenenfalls zusätzlich durch eine oder zwei Methoxygruppen.

(b) im Fall -A=B- = -N=CH-:

in Ii: durch eine oder zwei Hydroxy- und/oder Z-R-Gruppen;

in Ik: durch eine Alkenyloxy-, Alkinyloxy-, Cyanmethoxy-, Alkyloxycarbonylmethoxy-, Alkylthio- oder Alkylsulfinylgruppe und gegebenenfalls durch eine zusätzliche Hydroxy- Alkyloxy-, Alkenyloxy- oder Alkinyloxygruppe wobei die Alkyl-, Alkenyl- und Alkinylgruppen jeweils bis zu 3 C-Atome besitzen;

in Ik: durch eine Ethoxy-, propyloxy-, Isopropyloxy-, Allyloxy-, Propargyloxy-, 2-Hydroxyethoxy-, Cyanmethoxy-, Alkyloxycarbonylmethoxy-, Methylthio- oder Methylsulfinylgruppe und gegebenenfalls durch eine zusätzliche Hydroxy-, Alkyloxy-, Allyloxy- oder Propargyloxygruppe, wobei die Alkyloxygruppen jeweils 1 - 3 C-Atome besitzen;

in Ie: durch eine oder zwei Allyloxy-, Allylthio-, Propargyloxy- und/oder propargylthiogruppen und gegebenenfalls zusätzlich durch eine Alkyloxy- oder Alkylthiogruppe mit jeweils 1 - 3 C-Atomen;

in Im: durch eine Allyloxy- oder Allylthiogruppe und gegebenenfalls zusätzlich durch eine Methoxygruppe;

in In: durch eine Propargyloxy- oder propargylthiogruppe und gegebenenfalls zusätzlich durch eine Methoxygruppe;

in Io: durch eine Cyanmethoxy- oder Cyanmethylthiogruppe und gegebenenfalls zusätzlich durch eine Methoxygruppe;

in Ip: durch eine Methoxycarbonylmethoxy-, Ethoxycarbonylmethoxy-, Methoxycarbonylmethylthio- oder Ethoxycarbonylmethylthiogruppe und gegebenenfalls zusätzlich durch eine Methoxygruppe.

in Iq: durch eine Alkylthio-, Alkenylthio- oder Alkinylthiogruppe mit jeweils bis zu 3 C-Atomen.

Besonders bevorzugt sind diejenigen Verbindungen der Formeln I bzw. Ia bis Iq, worin die Substituenten in 2- und/ oder 4-Stellung stehen.

Gegenstand der Erfindung ist ferner ein Verfahren zur Herstellung der Verbindungen der Formel I sowie ihrer physiologisch unbedenklichen Salze, dadurch gekennzeichnet, daß man ein Diaminopyridin der allgemeinen Formel II

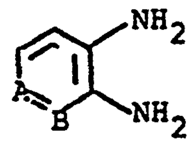

II

worin

die Gruppe -A=B- die oben angegebene Bedeutung hat mit einer Benzoesäure der allgemeinen Formel III

HOOC-Ar III

worin

Ar die oben angegebene Bedeutung hat

oder mit einem ihrer reaktionsfähigen Derivate

oder mit einem Aldehyd der allgemeinen Formel IV

OCH-Ar IV

worin

Ar die oben angegebene Bedeutung hat in Gegenwart eines Oxydationsmittels umsetzt

oder daß man eine Verbindung, die der Formel I entspricht, aber anstelle einer oder mehrerer Ethergruppen eine oder mehrere freie Hydroxygruppen enthält, mit einem Veretherungsmittel behandelt oder daß man eine Verbindung, die der Formel I entspricht, aber an Stelle einer oder mehrerer freier Hydroxy- und/oder Mercaptogruppen eine oder mehrere geschützte Hydroxy- oder Mercaptogruppen enthält, mit einem solvolysierenden oder hydrogenolysierenden Mittel behandelt

und daß man gegebenenfalls in dem erhaltenen Produkt Hydroxygruppen verethert und/oder Mercaptogruppen in Thioethergruppen überführt und/oder Thioethergruppen zu Sulfinylgruppen oxydiert

und/oder eine erhaltene Verbindung durch Behandeln mit einer Säure bzw. Base in eines ihrer physiologisch unbedenklichen Salze umwandelt.

Die Verbindungen der Formel I werden im übrigen nach an sich bekannten Methoden hergestellt, wie sie in der Literatur (z. B. in den Standardwerken wie Houben-Weyl, Methoden der Organischen Chemie, Georg-Thieme-Verlag, Stuttgart; insbesondere aber in der DE-OS 23 05 339) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Vorzugsweise werden die Verbindungen der Formel I erhalten durch Reaktion von II mit Benzoesäuren der Formel III oder ihren reaktionsfähigen Derivaten. Als reaktionsfähige Derivate eignen sich insbesondere die entsprechenden Nitrile, Säurehalogenide, Ester, Amide, Imidsäureester, Imidsäurethioester, Imidsäurehalogenide, Amidine, Thiocarbonsäureester, Dithiocarbonsäureester oder Orthoester.

Die Ausgangsstoffe II und III sind teilweise bekannt. Sofern sie nicht bekannt sind, können sie nach an sich bekannten Methoden hergestellt werden. So sind viele Benzoesäuren der Formel III beispielsweise durch Veretherung entsprechender Hydroxy-, Dihydroxy- oder Trihydroxybenzoesäuren erhältlich; diese Veretherung kann auch stufenweise durchgeführt werden.

Im einzelnen erfolgt die Umsetzung von II mit III in Gegenwart oder Abwesenheit eines inerten Lösungsmittels bei Temperaturen zwischen etwa -20 und etwa 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin, Pyridin oder Picoline; Glykole und Glykolether wie Ethylenglykol, Diethylenglykol, 2-Methoxy-ethanol; Ketone wie Aceton; Ether wie Tetrahydrofuran oder Dioxan; Amide wie Dimethylformamid; Sulfoxide wie Dimethylsulfoxid. Auch Gemische dieser Lösungsmittel sind geeignet. In manchen Fällen empfiehlt sich der Zusatz von katalytischen Mengen einer Säure wie p-Toluolsulfonsäure oder der Zusatz eines Dehydratisierungsmittels wie Phosphoroxychlorid, Polyphosphorsäure oder Thionylchlorid, wobei das Dehydratisierungsmittel auch als Lösungsmittel dienen kann.

Verwendet man die freien Benzoesäuren der Formel III, so wird die Reaktion zweckmäßig in Gegenwart eines der genannten Dehydratisierungsmittel und gegebenenfalls einer tertiären Base wie Pyridin oder Triethylamin durchgeführt, vorzugsweise bei Temperaturen zwischen -20 und 150°.

Die Umsetzung kann auch stufenweise durchgeführt werden. So ist es z. B. möglich, II mit einem Säurechlorid der Formel Ar-COCl partiell zum 2-Amino-3-ArCONH-pyridin, 3-Amino-4-ArCONH-pyridin bzw. zum 4-Amino-3-ArCONH-pyridin (oder zu einem Gemisch der beiden letztgenannten Isomeren) zu acylieren, das anschließend, z.B. mit $POCl_3$, zu I dehydratisiert wird.

Es ist auch möglich, an Stelle von III einen entsprechenden Aldehyd der Formel IV zu verwenden, wenn man gleichzeitig in Gegenwart eines Oxydationsmittels arbeitet. Bevorzugt verwendet man als Oxydationsmittel

Schwefel in einem Kohlenwasserstoff wie Benzol, Toluol, Xylol oder Mesitylen oder Natriumdisulfit in Lösungsmitteln wie Dimethylacetamid, jeweils bei Temperaturen zwischen etwa 80 und etwa 200°. Diese Reaktionsvariante eignet sich besonders zur Herstellung von Verbindungen der Formel I, die Gruppen (z.B Alkyloxycarbonylgruppen) enthalten, die gegenüber bestimmten Dehydratisierungsmitteln (z.B. $POCl_3$) nicht völlig inert sind. Die Aldehyde der Formel IV sind in der Regel neu und beispielsweise durch Veretherung der entsprechenden Hydroxyaldehyde erhältlich.

Die Verbindungen der Formel I, insbesondere diejenigen der Formel Ia,sind weiterhin erhältlich, indem man eine sonst der Formel I entsprechende Verbindung, die jedoch an Stelle einer (oder mehrerer) Ethergruppen eine (oder mehrere) freie Hydroxygruppe(n) enthält, verethert.

Geeignete Ausgangsstoffe für diese Veretherung entsprechen z.B. der allgemeinen Formel V

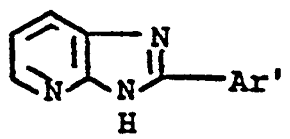

V

worin

Ar' einen Phenylrest bedeutet, der durch eine Hydroxygruppe substituiert ist und zusätzlich durch eine bis drei Alkinyloxygruppen und/oder eine oder zwei Cyanmethoxy-, Alkyloxycarbonylmethoxy-, Hydroxy-, Alkyloxy- und/oder Alkenyloxygruppen substituiert sein kann, jedoch höchstens vierfach substitituiert ist.

Als Veretherungsmittel eignen sich vorzugsweise solche der allgemeinen Formel VI

X - R VI

worin

X Cl, Br, J, OH, Alkylsulfonyloxy oder Arylsulfonyloxy und

R Alkinyl, Cyanmethyl, Alkyloxycarbonylmethyl, Alkyl oder Alkenyl bedeuten,

wobei die Alkyl-, Alkenyl- und Alkinylgruppen jeweils bis zu 5 und die Arylgruppe 6 - 10 C-Atome besitzen.

Typische Veretherungsmittel sind z.B. Propargylchlorid oder -bromid, Chlor- oder Bromacetonitril, Chlor- oder Bromessigsäure- methyl- und ethylester, Methyl-, Ethyl-, Propyl-, Isopropyl- und Allylchlorid oder -bromid.

Zur Veretherung wird die Hydroxyverbindung V zweckmäßig zunächst in eines ihrer Salze, z.B. das Na-Salz, umgewandelt, das anschließend mit VI in einem der angegebenen Lösungsmittel bei Temperaturen zwischen etwa 0 und etwa 120° umgesetzt wird. Man kann auch die freie Hydroxyverbindung V mit dem entsprechenden Alkohol VI (X = OH) in Gegenwart von Diethyl-azodicarboxylat/Triphenylphosphin verethern, zweckmäßig in einem Lösungsmittel wie Tetrahydrofuran oder Dioxan bei Temperaturen zwischen etwa 10 und etwa 40°.

Verbindungen der Formel I, insbesondere diejenigen der Formel Ib, die freie Hydroxy- und/oder Mercaptogruppen enthalten, sind weiterhin durch Solvolyse oder Hydrogenolyse von entsprechenden Verbindungen erhältlich, in denen die Hydroxy- und/oder Mercaptogruppen durch Schutzgruppen blockiert sind, welche auf diese Weise abgespalten werden können.

Die Ausgangsstoffe für diese Solvolyse bzw. Hydrogenolyse entsprechen der allgemeinen Formel VII

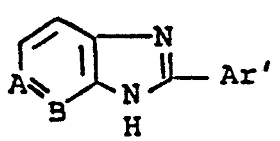

VII

worin

Ar' einen Phenylrest bedeutet, der im Fall (a) (-A=B- = -CH=N-) durch eine oder zwei geschützte Hydroxygruppen sowie durch eine oder zwei Alkinyloxy-, Cyanmethoxy- und/oder Alkyloxycarbonylmethoxygruppen substituiert ist und durch eine zusätzliche Hydroxy-, Alkyloxy-, Alkenyloxy- oder Alkinyloxygruppe substituiert sein kann, insgesamt jedoch höchstens vierfach substituiert ist, oder der

in Fall (b) (-A=B- = -N=CH-)

durch eine bis drei geschützte Hydroxy- und/oder Mercaptogruppen substituiert ist und zusätzlich eine oder zwei freie Hydroxy- und/oder Mercapto- und/oder Z-R-Gruppen (wobei Z und R die angegebene Bedeutung

haben) tragen kann, insgesamt jedoch höchstens dreifach substituiert ist, worin jedoch der Phenylrest nur dann durch geschützte Hydroxy- oder durch Methoxygruppen substituiert ist, wenn er gleichzeitig noch mindestens einen anderen jeweils davon verschiedenen Substituenten trägt.

Die Ausgangsstoffe der Formel VII sind beispielsweise erhältlich durch Reaktion von II mit Benzoesäuren der Formel HOOC-Ar' oder ihren reaktionsfähigen Derivaten nach den oben angegebenen Methoden.

Als Schutzgruppen kommen die bekannten Hydroxyschutzgruppen und Mercaptoschutzgruppen in Frage. Diese Ausdrücke beziehen sich auf Gruppen, die geeignet sind, Hydroxy- bzw. Mercaptogruppen vor chemischen Umsetzungen zu schützen, die aber leicht entfernbar sind, nachdem die gewünschte chemische Reaktion an einer anderen Stelle des Moleküls durchgeführt worden ist. Typische Schutzgruppen sind beispielsweise leicht spaltbare Ester-, Thioester-, Ether- und Thioethergruppen mit vorzugsweise 1 - 12 C-Atomen, im einzelnen z. B. Alkanoyl mit vorzugsweise 1 - 6 C-Atomen wie Acetyl, Aroyl mit vorzugsweise 7 - 11 C-Atomen wie Benzoyl, unsubstituiertes.und substituiertes Aryl und Aralkyl mit jeweils bis zu 11 C-Atomen wie 2,4-Dinitrophenyl, Benzyl, Triphenylmethyl, ferner z. B. Tetrahydropyranyl. Natur und Größe der Schutzgruppen sind nicht kritisch, da sie nach dem vorliegenden Verfahren abgespalten werden.

Eine Solvolyse der Schutzgruppen gelingt vorzugsweise in Form der Hydrolyse in wässerigem oder wässerigalkoholischem Medium in Gegenwart von Säuren wie Salzsäure oder von Basen wie Natrium- oder Kaliumhydroxid bei Temperaturen zwischen etwa 0 und 100°.

Eine Hydrogenolyse der hydrogenolytisch abspaltbaren Schutzgruppen gelingt z. B. in Gegenwart eines Schwermetallkatalysators wie Platin, Palladium oder Nickel in einem inerten Lösungsmittel wie Methanol, Ethanol, Tetrahydrofuran oder Ethylacetat bei Temperaturen Zwischen etwa 0 und 100° und Drucken zwischen etwa 1 und 200 bar.

Gewünschtenfalls kann man eine oder mehrere in dem erhaltenen Produkt vorhandene Hydroxygruppe(n) verethern und/oder Mercaptogruppe(n) in Thioethergruppen überführen.

Als Veretherungsmittel (bzw. Thioveretherungsmittel) eignen sich dafür vorzugsweise solche der oben genannten Formel X-R (VI), worin X und R die angegebenen Bedeutungen haben, worin jedoch R außerdem Hydroxyalkyl mit bis zu 5 C-Atomen bedeuten kann.

Typische Veretherungsmittel (bzw. Thioveretherungsmittel) sind z.B. die oben einzeln genannten, ferner 2-Hydroxyethylchlorid oder -bromid.

Die Veretherung (bzw. Thioveretherung) erfolgt zweckmäßig unter den oben für die Veretherung von V angegebenen Bedingungen.

Ferner kann man, falls erwünscht, eine in einem erhaltenen Produkt der Formel I gegebenenfalls vorhandene Thioethergruppe zu einer Sulfinylgruppe oxydieren, zweckmäßig mit Wasserstoffperoxid, Persäuren oder Cr(VI)-Verbindungen wie Chromsäure in Gegenwart eines inerten Lösungsmittels wie Wasser, einen Alkohol (z. B. Methanol oder Ethanol), einer Säure (z. B. Essigsäure) oder einen Keton (z. B. Aceton) bei Temperaturen zwischen etwa -20 und 100°.

Eine Base der Formel I kann mit einer Säure in das zugehörige Säureadditionssalz überführt werden. Für diese Umsetzung kommen Säuren in Frage, die physiologisch unbedenkliche Salze liefern. So können anorganische Säuren vwewendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische einoder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Apfelsäure, Benzoesäure, Salicylsäure, 2-oder 3-Phenylpropionsäure, Citronensäure, Gluconsäure, Ascorbinsäure, Nicotinsäure, Isonicotinsäure, Methanoder Ethansulfonsäure, Ethandisulfonsäure, 2-Hydroxy-ethansulfonsäure, Benzolsulfonsäure, p-Toluolsulfonsäure, Naphthalin-mono- und disulfonsäuren, Laurylschwefelsäure.

Eine Säure der Formel I kann durch Umsetzung mit einer Base in eines ihrer physiologisch unbedenklichen Metall- bzw. Ammoniumsalze übergeführt werden. Als Salze kommen insbesondere die Natrium-, Kalium-, Magnesium-, Calcium- und Ammoniumsalze in Betracht, ferner substituierte Ammoniumsalze, z. B. die Dimethyl-, Diethyloder Dii-sopropylammonium-, Monoethanol-, Diethanolund Triethanolammonium-, Cyclohexylammonium-, Dicyclohexylammonium- und Dibenzylethylendiammoniumsalze.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder

Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und oder Hilfsstoffe wie Gleit-, Konservierungs-, Stabilisrerungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I können bei der Bekämpfung von Krankheiten, insbesondere von Herzinsuffizienz, sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen, wie Sulmazol oder Amrinon, verabreicht, vorzugsweise in Dosierungen zwischen etwa 10 und 900 mg, insbesondere zwischen 20 und 100 mg pro Dosierungseinheit. Die tägliche Dosierung liegt vorzugsweise zwischen etwa 0,2 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

**Beispiel 1**

Ein Gemisch von 10,9 g 2,3-Diaminopyridin (IIa) und 20,6 g 2-Methoxy-4-propargyloxy-benzoesäure [F. 142°; erhältlich durch Reaktion von 2,4-Dihydroxy-benzoesäure-methylester mit propargylchlorid zu 2-Hydroxy-4-propargyloxy-benzoesäuremethylester (F. 100°), Reaktion mit Methyljodid zu 2-Methoxy-4-propargyloxy-benzoesäuremethylester (F. 88°) und Verseifung] wird portionsweise unter Rühren zu 500 ml $POCl_3$ gegeben. Man kocht 4 Stunden, dampft ein und versetzt den Rückstand mit 375 ml 10%iger Salzsäure. Das ausgefallene 2-(2-Methoxy-4-propargyloxy-phenyl)-imidazo(4,5-b)pyridin-hydrochlorid ("M") wird abfiltriert. F. 247° (aus Methanol).

Analog erhält man aus IIa bzw. 3,4-Diaminopyridin (IIb) und den entsprechenden Benzoesäuren der Formel III (z.B. o-Propargyloxybenzoesäure, F. 82°; m-Propargyloxybenzoesäure, F. 130° p-Propargyloxybenzoesäure, F. 224°; 2-Methoxy-5-propargyloxy-benzoesäure, F. 103°; 3-Methoxy-4-propargyloxy-benzoesäure, F.195° 3,5-Dimethoxy-4-propargyloxy-benzoesäure, F.208°; 2,4-Bis-propargyloxy-benzoesäure, F. 152°), wobei man die Reaktionstemperatur zwischen 70 und 110 halten kann:

2-(p-Ethinyloxyphenyl)-imidazo(4,5-b)pyridin
2-(o-Propargyloxyphenyl)-imidazo(4,5-b)pyridin, Hydrochlorid, F. 223° 2-(m-Propargyloxyphenyl)-imidazo(4,5-b)pyridin, Hydrochlorid, F. 195°
2-(p-Propargyloxyphenyl)-imidazo(4,5-b)pyridin, F.218°
2-(p-3-Pentin-1-yloxy-phenyl)-imidazo(4,5-b)pyridin
2-(o-Cyanmethoxyphenyl)-imidazo(4,5-b)pyridin
2-(m-Cyanmethoxyphenyl)-imidazo(4,5-b)pyridin
2-(p-Cyanmethoxyphenyl)-imidazo(4,5-b)pyridin
2-(o-Methoxycarbonylmethoxyphenyl)-imidazo(4,5-b)-pyridin
2-(m-Methoxycarbonylmethoxyphenyl)-imidazo(4,5-b)-pyridin
2-(p-Methoxycarbonylmethoxyphenyl)-imidazo(4,5-b)-pyridin
2-(o-Ethoxycarbonylmethoxyphenyl)-imidazo(4,5-b)-pyridin
2-(m-Ethoxycarbonylmethoxyphenyl)-imidazo(4,5-b)-pyridin
2-(p-Ethoxycarbonylmethoxyphenyl)-imidazo(4,5-b)-pyridin, Hemifumarat, F. 158°
2-(p-Propyloxvcarbonylmethoxyphenyl)-imidazo(4,5-b)-pyridin
2-(p-Butyloxycarbonylmethoxyphenyl)-imidazo(4,5-b)-pyridin
2-(p-Pentyloxycarbonylmethoxyphenyl)-imidazo(4,5-b)-pyridin
2-(2-Hydroxy-4-propargyloxy-phenyl)-imidazo(4,5-b)-pyridin
2-(3-Hydroxy-4-propargyloxy-phenyl)-imidazo(4,5-b)-pyridin
2-(4-Hydroxy-2-propargyloxy-phenyl)-imidazo(4,5-b)-pyridin
2-(2-Methoxy-3-propargyloxy-phenyl)-imidazo(4,5-b)-pyridin
2-(2-Methoxy-5-propargyloxy-phenyl)-imidazo(4,5-b)-pyridin, Hydrochlorid, F. 262°
2-(2-Methoxy-6-propargyloxy-phenyl)-imidazo(4,5-b)-pyridin

2-(3-Methoxy-2-propargyloxy-phenyl)-imidazo(4,5-b)-pyridin
2-(3-Methoxy-4-propargyloxy-phenyl)-imidazo(4,5-b)-pyridin, Hydrochlorid, F. 238°
2-(3-Methoxy-5-propargyloxy-phenyl)-imidazo(4,5-b)-pyridin
2-(3-Methoxy-6-propargyloxy-phenyl)-imidazo(4,5-b)-pyridin
2-(4-Methoxy-2-propargyloxy-phenyl)-imidazo(4,5-b)-pyridin, Fumarat, F. 220°
2-(4-Methoxy-3-propargyloxy-phenyl)-imidazo(4,5-b)-pyridin
2-(2-Ethoxy-3-propargyloxyphenyl)-imidazo(4,5-b)-pyridin
2-(2-Ethoxy-4-propargyloxyphenyl)-imidazo(4,5-b)-pyridin
2-(2-Ethoxy-5-propargyloxyphenyl)-imidazo(4,5-b)-pyridin
2-(2-Ethoxy-6-propargyloxyphenyl)-imidazo(4,5-b)-pyridin
2-(3-Ethoxy-2-propargyloxyphenyl)-imidazo(4,5-b)-pyridin
2-(3-Ethoxy-4-propargyloxyphenyl)-imidazo(4,5-b)-pyridin
2-(3-Ethoxy-5-propargyloxyphenyl)-imidazo(4,5-b)-pyridin
2-(4-Ethoxy-2-propargyloxyphenyl)-imidazo(4,5-b)-pyridin
2-(4-Ethoxy-3-propargyloxyphenyl)-imidazo(4,5-b)-pyridin
2-(3,5-Dimethoxy-4-propargyloxy-phenyl)-imidazo-(4,5-b)-pyridin, Hydrochlorid, F. 250°
2-(3,5-Diethoxy-4-propargyloxyphenyl)-imidazo-(4,5-b)-pyridin
2-(2-Allyloxy-4-propargyloxyphenyl)-imidazo(4,5-b)-pyridin
2-(3-Allyloxy-4-propargyloxyphenyl)-imidazo(4,5-b)-pyridin
2-(4-Allyloxy-2-propargyloxyphenyl)-imidazo(4,5-b)-pyridin
2-(2,4-Bis-propargyloxyphenyl)-imidazo(4,5-b)-pyridin, Hemifumarat, F. 210°
2-(4-Cyanmethoxy-2-hydroxyphenyl)-imidazo(4,5-b)-pyridin
2-(4-Cyanmethoxy-2-methoxyphenyl)-imidazo(4,5-b)-pyridin, Hemifumarat, F. 212°
2-(4-Cyanmethoxy-2-ethoxyphenyl)-imidazo(4,5-b)-pyridin
2-(2-Allyloxy-4-cyanmethoxyphenyl)-imidazo(4,5-b)-pyridin
2-(2,4-Bis-cvanmethoxyphenyl)-imidazo(4,5-b)-pyridin
2-(2-Cyanmethoxy-4-prcpargyloxyphenyl)-imidazo-(4,5-b)pyridin
2-(4-Cyanmethoxy-2-propargyloxyphenyl)-imidazo-(4,5-b)pyridin
2-(4-Methoxycarbonylmethoxy-2-hydroxyphenyl)-imidazo(4,5-b)pyridin
2-(4-Methoxycarbonylmethoxy-2-methoxyphenyl)-imidazo(4,5-b)pyridin
2-(2-Ethoxy-4-methoxycarbonylmethoxyphenyl)-imidazo(4,5-b)pyridin
2-(2-Allyloxy-4-methoxycarbonvlmethoxyphenyl)-imidazo(4,5-b)pyridin
2-(2,4-Bis-methoxycarbonylmethoxyphenyl)-imidazo(4,5-b)pyridin
2-(2-Methoxycarbonylmethoxy-4-propargyloxyphenyl)-imidazo(4,5-b)pyridin
2-(4-Methoxycarbonylmethoxy-2-propargyloxyphenyl)-imidazo(4,5-b)pyridin
2-(4-Ethoxycarbonylmethoxy-2-hydroxyphenyl)-imidazo(4,5-b)pyridin
2-(4-Ethoxycarbonylmethoxy-2-methoxyphenyl)-imidazo(4,5-b)pyridin, Hemifumarat, F. 195°
2-(2-Ethoxy-4-ethoxycarbonylmethoxyphenyl)-imidazo(4,5-b)pyridin
2-(2-Allyloxy-4-ethoxycarbonylmethoxyphenyl)-imidazo(4,5-b)pyridin
2-(2,4-Bis-ethoxycarbonylmethoxyphenyl)-imidazo(4,5-b)pyridin
2-(2-Ethoxycarbonylmethox-y-4-propargyloxyphenyl)-imidazo(4,5-b)pyridin
2-(4-Ethoxycarbonylmethoxy-2-propargyloxyphenyl)-imidazo(4,5-b)pyridin
2-(o-Ethoxyphenyl)-imidazo(4,5-c)pyridin
2-(m-Ethoxyphenyl)-imidazo(4,5-c)pyridin
2-(p-Ethoxyphenyl)-imidazo(4,5-c)pyridin
2-(o-propylcxyphenyl)-imidazo(4,5-c)pyridin
2-(m-propyloxyphenyl)-imidazo(4,5-c)pyridin
2-(p-Propyloxyphenyl)-imidazo(4,5-c)pyridin
2-(o-Isopropyloxyphenyl)-imidazo(4,5-c)pyridin
2-(m-Isopropyloxyphenyl)-imidazo(4,5-c)pyridin
2-(p-Isopropyloxvphenyl)-imidazo(4,5-c)pyridin
2-(o-Allyloxyphenyl)-imidazo(4,5-c)pyridin, Hydrochlorid, F. 212°
2-(m-Allyloxyphenyl)-imidazo(4,5-c)pyridin
2-(p-Allyloxyphenyl)-imidazo(4,5-c)pyridin, Fumarat, F. 215°
2-(p-Ethinyloxyphenyl)-imidazo(4,5-c)pyridin
2-(o-Propargyloxyphenyl)-imidazo(4,5-c)pyridin, Fumarat, F, 192°
2-(m-Propargyloxyphenyl)-imidazo(4,5-c)pyridin. Hydrochlorid, F. 250°
2-(p-Propargyloxyphenyl)-imidazo(4,5-c)pyridin, Hemifumarat, F. 230°
2-(p-3-Pentin-1-yloxy-phenyl)-imidazo(4,5-c)pyridin
2-(o-2-Hydroxyethoxyphenyl)-imidazo(4,5-c)pyridin
2-(m-2-Hydroxyethoxyphenyl)-imidazo(4,5-c)pyridin
2-(p-2-Hydroxyethoxyphenyl)-imidazo(4,5-c)pyridin
2-(o-Cyanmethoxyphenyl)-imidazo(4,5-c)pyridin
2-(m-Cyanmethoxyphenyl)-imidazo(4,5-c)pyridin
2-(p-Cyanmethoxyphenyl)-imidazo(4,5-c)pyridin

2-(o-Methoxycarbonylmethoxyphenyl)-imidazo(4,5-c)-pyridin
2-(m-Methoxycarbonylmethoxyphenyl) - imidazo (4, 5-c)-pyridin
2-(p-Methoxycarbonylmethoxyphenyl)-imidazo(4,5-c)-pyridin
2-(o-Ethoxycarbonylnethoxyphenyl)-imidazo(4,5-c)-pyridin
2-(m-Ethoxycarbonylmethoxyphenyl)-imidazo(4,5-c)-pyridin
2-(p-Ethoxycarbonylmethoxyphenyl)-imidazo(4,5-c)-pyridin
2-(p-Propyloxycarbonylmethoxyphenyl)-imidazo(4,5-c)-pyridin
2-(p-Butyloxycarbonylmethoxyphenyl)-imidazo(4,5-c)-pyridin
2-(p-pentyloxycarbonylmethoxyphenyl)-imidazo(4,5-c) pyridin
2-(p-Mercaptophenyl)-imidazo(4,5-c)pyridin
2-(o-Methylthio-phenyl)-imidazo(4,5-c)pyridin, Hydrochlorid, F. 230°
2-(m-Methylthio-phenyl)-imidazo(4,5-c)pyridin
2-(p-Methylthio-phenyl)-imidazo(4,5-c)pyridin
2-(o-Ethylthio-phenyl)-imidazo(4,5-c)pyridin
2-(m-Ethylthio-phenyl)-imidazo(4,5-c)pyridin
2-(p-Ethylthio-phenyl)-imidazo(4,5-c)pyridin
2-(o-Propylthio-phenyl)-imidazo(4,5-c)pyridin
2-(m-Propylthio-phenyl)-imidazo(4,5-c)pyridin
2-(p-Propylthio-phenyl)-imidazo(4,5-c)pyridin
2-(o-Allylthio-phenyl)-imidazo(4,5-c)pyridin
2-(m-Allylthio-phenyl)-imidazo(4,5-c)pyridin
2-(p-Allylthio-phenyl)-imidazo(4,5-c)pyridin
2-(o-Propargylthio-phenyl)-imidazo(4,5-c)pyridin F. 248°
2-(m-Propargylthio-phenyl)-imidazo(4 5-c)pyridin
2-(p-Propargylthio-phenyl)-imidazo(4,5-c)pyridin
2-(o-2-Hydroxyethylthio-phenyl)-imidazo(4,5-c)-pyridin
2-(m-2-Hydroxyethylthio-phenyl)-imidazo(4,5-c)-pyridin
2-(p-2-Hydroxyethylthio-phenyl)-imidazo(4,5-c)-pyridin
2-(o-Cyanmethylthio-phenyl)-imidazo(4,5-c)pyridin
2-(m-Cyanmethylthio-phenyl)-imidazo(4,5-c)pyridin
2-(p-Cyanmethylthio-phenyl)-imidazo(4,5-c)pyridin
2-(o-Methoxycarbonylmethylthio-phenyl)-imidazo(4,5-c)-pyridin
2-(m-Methoxycarbonylmethylthio-phenyl)-imidazo(4,5-c)-pyridin
2-(p-Methoxycarbonylmethylthio-phenyl)-imidazo(4,5-c)-pyridin
2-(o-Ethoxycarbonylmethylthio-phenyl)-imidazo(4,5-c)-pyridin
2-(m-Ethoxycarbonylmethylthio-phenyl)-imidazo(4,5-c)-pyridin
2-(p-Ethoxycarbonylmethylthio-phenyl)-imidazo(4,5-c)-pyridin
2-(o-Methylsulfinylphenyl)-imidazo(4,5-c)pyridin, Hydrochlorid, F. 230°
2-(m-Methylsulfinylphenyl)-imidazo(4,5-c)pyridin
2-(p-Methylsulfinylphenyl)-imidazo(4,5-c)pyridin
2-(o-Ethylsulfinylphenyl)-imidazo(4,5-c)pyridin
2-(m-Ethylsulfinylphenyl)-imidazo(4,5-c)pyridin
2-(p-Ethylsulfinylphenyl)-imidazo(4,5-c)pyridin
2-(o-Propylsulfinylphenyl)-imidazo(4,5-c)pyridin
2-(m-Propylsulfinylphenyl)-imidazo(4,5-c)pyridin
2-(p-Propylsulfinylphenyl)-imidazo(4,5-c)pyridin
2-(o-Allylsulfinylphenyl)-imidazo(4,5-c)pyridin
2-(m-Allylsulfinylphenyl)-imidazo(4,5-c)pyridin
2-(p-Allylsulfinylphenyl)-imidazo(4,5-c)pyridin
2-(o-Propargylsulfinylphenyl)-imidazo(4,5-c)pyridin
2-(m-Propargylsulfinylphenyl)-imidazo(4,5-c)pyridin
2-(p-Propargylsulfinylphenyl)-imidazo(4,5-c)pyridin
2-(o-2-Hydroxyethylsulfinylphenyl)-imidazo(4,5-c)-pyridin
2-(m-2-Hydroxyethylsulfinylphenyl)-imidazo(4,5-c)-pyridin
2-(p-2-Hydroxyethylsulfinylphenyl)-imidazo(4,5-c)-pyridin
2-(o-Cyanmethylsulfinylphenyl)-imidazo(4,5-c)pyridin
2-(m-Cyanmethylsulfinylphenyl)-imidazo(4,5-c)pyridin
2-(p-Cyanmethylsulfinylphenyl)-imidazo(4,5-c)pyridin
2-(o-Methoxycarbonylmethylsulfinylphenyl)-imidazo-(4,5-c)pyridin
2-(m-Methoxycarbonylmethylsulfinylphenyl)-imidazo-(4,5-c)pyridin
2-(p-Methoxycarbonylmethylsulfinylphenyl)-imidazo-(4,5-c)pyridin
2-(o-Ethoxycarbonylmethylsulfinylphenyl)-imidazo-(4,5-c)pyridin
2-(m-Ethoxycarbonylmethylsulfinylphenyl)-imidazo-(4,5-c)pyridin
2-(p-Ethoxycarbonylmethylsulfinylphenyl)-imidazo-(4,5-c)pyridin
2-(2-Hydroxy-4-methoxyphenyl)-imidazo(4,5-c)pyridin

2-(4-Hydroxy-2-methoxyphenyl)-imidazo(4,5-c)pyridin, Fumarat, F. 255°
2-(2-Ethoxy-4-hydroxyphenyl)-imidazo(4,5-c)pyridin
2-(4-Ethoxy-2-hydroxyphenyl)-imidazo(4,5-c)pyridin
2-(2-Ethoxy-4-methoxyphenyl)-imidazo(4,5-c)pyridin, Fumarat, F. 198°
2-(4-Ethoxy-2-methoxyphenyl)-imidazo(4,5-c)pyridin, Fumarat, F. 213°
2-(2,4-Diethoxyphenyl)-imidazo(4,5-c)pyridin
2-(2-Isopropyloxy-4-methoxyphenyl)-imidazo(4,5-c)-pyridin
2-(4-Isopropyloxy-2-methoxyphenyl)-imidazo(4,5-c) pyridin, Bis-Fumarat, F. 220°
2-(2,4-Diisopropyloxyphenyl)-imidazo(4,5-c)pyridin
2-(2-Allyloxy-4-hydroxyphenyl)-imidazo(4,5-c)pyridin
2-(4-Allyloxy-2-hydroxyphenyl)-imidazo(4,5-c)pyridin.
2-(2-Allyloxy-4-methoxyphenyl)-imidazo(4,5-c)pyridin, Hydrochlorid, F. 206°
2-(4-Allyloxy-2-methoxyphenyl)-imidazo(4,5-c)pyridin, Hydrochlorid, F. 250°
2-(2-Allyloxy-4-ethoxyphenyl)-imidazo(4,5-c)pyridin
2-(4-Allyloxy-2-ethoxyphenyl)-imidazo(4,5-c)pyridin
2-(2-Allyloxy-4-isopropoxyphenyl)-imidazo(4,5-c)-pyridin
2-(4-Allyloxy-2-isopropoxyphenyl)-imidazo(4,5-c)-pyridin
2-(2,4-Bis-allyloxyphenyl)-imidazo(4,5-c)pyridin, Hydrochlorid, F. 179°
2-(2-Hydroxy-4-propargyloxyphenyl)-imidazo(4,5-c)-pyridin
2-(3-Hydroxy-4-propargyloxyphenyl)-imidazo(4,5-c) pyridin
2-(4-Hydroxy-2-propargyloxyphenyl)-imidazo(4,5-c)-pyridin
2-(2-Methoxy-3-propargyloxy-phenyl)-imidazo(4,5-c)-pyridin, Hydrochlorid, F. 242°
2-(2-Methoxy-4-propargyloxy-phenyl)-imidazo(4,5-c)-pyridin
2-(2-Methoxy-5-propargyloxy-phenyl)-imidazo(4,5-c)-pyridin, Fumarat, F. 210°
2-(2-Methoxy-6-propargyloxy-phenyl)-imidazo(4,5-c)-pyridin
2-(3-Methoxy-2-propargyloxy-phenyl)-imidazo(4,5-c)-pyridin.
2-(3-Methoxy-4-propargyloxy-phenyl)-imidazo(4,5-c)-pyridin
2-(3-Methoxy-5-propargyloxy-phenyl)-imidazo(4,5-c)-pyridin
2-(3-Methoxy-6-propargyloxy-phenyl)-imidazo(4,5-c)-pyridin
2-(4-Methoxy-2-propargyloxy-phenyl)-imidazo(4,5-c)-pyridin, Fumarat, F. 210°
2-(4-Methoxy-3-propargyloxy-phenyl)-imidazo(4,5-c)-pyridin
2-(2-Ethoxy-3-propargyloxyphenyl)-imidazo(4,5-c)-pyridin
2-(2-Ethoxy-4-propargyloxyphenyl)-imidazo(4,5-c)-pyridin
2-(2-Ethoxy-5-propargyloxyphenyl)-imidazo(4,5-c)-pyridin
2-(2-Ethoxy-6-propargyloxyphenyl)-imidazo(4,5-c)-pyridin
2-(3-Ethoxy-2-propargyloxyphenyl)-imidazo(4,5-c)-pyridin
2-(3-Ethoxy-4-propargyloxyphenyl)-imidazo(4,5-c)-pyridin
2-(3-Ethoxy-5-propargyloxyphenyl)-imidazo(4,5-c)-pyridin
2-(4-Ethoxy-2-propargyloxyphenyl)-imidazo(4,5-c)-pyridin
2-(4-Ethoxy-3-propargyloxyphenyl)-imidazo(4,5-c)-pyridin
2-(3,5-Dimethoxy-4-propargylcxy-phenyl)-imidazo-(4,5-c)-pyridin; Dihydrochlorid, F. 238°
2-(3,5-Diethoxy-4-propargyloxyphenyl)-imidazo-(4,5-c)-pyridin
2-(2-Allyloxy-4-propargyloxyphenyl)-imidazo(4,5-c)-pyridin
2-(3-Allyloxy-4-propargyloxyphenyl)-imidazo(4,5-c)-pyridin
2-(4-Allyloxy-2-propargyloxyphenyl)-imidazo(4,5-c)-pyridin
2-(2,4-Bis-propargyloxyphenyl)-imidazo(4,5-c)-pyridin, Fumarat, F. 195°
2-/2-Hydroxy-4-(2-hydroxyethoxy)-phenyl]-imidazo-(4,5-c)pyridin
2-/4-Hydroxy-2-(2-hydroxyethoxy)-phenyl]-imidazo-(4,5-c)pyridin
2-[2-(2-Hydroxyethoxy)-4-methoxyphenyl]-imidazo-(4,5-c)pyridin, Hydrochlorid, F. 250°
2-[4-(2-Hydroxyethoxy)-4-methoxyphenyl]-imidazo-(4,5-c)pyridin
2-[2-Ethoxy-4-(2-hydroxyethoxy)-phenyl]-imidazo-(4,5-c)pyridin
2-[4-Ethoxy-2-(2-hydroxyethoxy)-phenyl]-imidazo-(4,5-c)pyridin
2-[2-Allyloxy-4-(2-hydroxyethoxy)-phenyl]-imidazo-(4,5-c)pyridin
2-[4-Allyloxy-2-(2-hydroxyethoxy)-phenyl]-imidazo-(4,5-c)pyridin
2-[2-(2-Hydroxyethoxy)-4-propargyloxyphenyl]-imidazo(4,5-c)pyridin
2-[4-(2-Hydroxyethoxy)-2-proparoyloxyphenyl]-imidazo(4,5-c)pyridin
2-[2,4-Bis-(2-hydroxyethoxy)-phenyl]-imidazo(4,5-c)-pyridin
2-(2-Cyanmethoxy-4-hydroxyphenyl)-imidazo(4,5-c)-pyridin
2-(4-Cyanmethoxy-2-hydroxyphenyl)-imidazo(4,5-c)-pyridin
2-(2-Cyanmethoxy-4-methoxyphenyl)-imidazo(4,5-c)-pyridin
2-(4-Cyanmethoxy-2-methoxyphenyl)-imidazo(4,5-c)-pyridin, Fumarat, F. 198°
2-(2-Cyanmethoxy-4-ethoxyphenyl)-imidazo(4,5-c)-pyridin
2-(4-Cyanmethoxy-2-ethoxyphenyl)-imidazo(4,5-c)-pyridin
2-(2-Allyloxy-4-cyanmethoxyphenyl)-imidazo(4,5-c)-pyridin
2-(4-Allyloxy-2-cyanmethoxyphenyl)-imidazo(4,5-c)-. pyridin

2-(2-Cyanmethoxy-4-propargyloxyphenyl)-imidazo-(4,5-c)pyridin
2-(4-Cyanmethoxy-2-propargyloxyphenyl)-imidazo-(4,5-c)pyridin
2-[2-Cyanmethoxy-4-(2-hydroxyethoxy)-phenyl]-imidazo(4,5-c)pyridin
2-[4-Cyanmethoxy-2-(2-hydroxyethoxy)-phenyl]-imidazo(4,5-c)pyridin
2-(2,4-Bis-cyanmethoxyphenyl)-imidazo(4,5-c)pyridin
2-(4-Methoxycarbonylmethoxy-2-hydroxyphenyl)-imidazo(4,5-c)pyridin
2-(4-Methoxycarbonylmethoxy-2-methoxyphenyl)-imidazo(4,5-c)pyridin
2-(2-Ethoxy-4-methoxycarbonylmethoxyphenyl)-imidazo(4,5-c)pyridin
2-(2-Allyloxy-4-methoxycarbonvlmethoxyphenyl)-imidazo(4,5-c)pyridin
2-(2,4-Bis-methoxycarbonylmethoxyphenyl)-imidazo(4,5-c)pyridin
2-(2-Methoxycarbonylmethoxy-4-propargyloxyphenyl)-imidazo(4,5-c)pyridin
2-(4-Methoxycarbonylmethoxy-2-propargyloxyphenyl)-imidazo(4,5-c)pyridin
2-(4-Ethoxycarbonylmethoxy-2-hydroxyphenyl)-imidazo(4,5-c)pyridin
2-(4-Ethoxycarbonylmethoxy-2-methoxyphenyl)-imidazo(4,5-c)pyridin
2-(2-Ethoxy-4-ethoxycarbonylmethoxyphenyl)-imidazo(4,5-c)pyridin
2-(2-Allyloxy-4-ethoxycarbonylmethoxyphenyl)-imidazo(4,5-c)pyridin
2-(2,4-Bis-ethoxycarbonylmethoxyphenyl)-imidazo(4,5-c)pyridin
2-(2-Ethoxycarbonylmethoxy-4-propargyloxyphenyl)-imidazo(4,5-c)pyridin
2-(4-Ethoxycarbonylmethoxy-2-propargyloxyphenyl)-imidazo(4,5-c)pyridin
2-(4-Mercapto-2-methoxy-phenyl)-imidazo(4,5-c)pyridin
2-(2-Mercapto-4-propargyloxyphenyl)-imidazo(4,5-c)-pyridin
2-(2-Hydroxy-4-methylthiophenyl)-imidazo(4,5-c)pyridin
2-(4-Hydroxy-2-methylthiophenyl)-imidazo(4,5-c)pyridin
2-(2-Methoxy-4-methylthiophenyl)-imidazo(4,5-c)pyridin, Hydrochlorid, F. 253°
2-(2-Methoxy-5-methylthiophenyl)-imidazo(4,5-c)pyridin, Hydrochlorid, F. 242°.
2-(4-Methoxy-2-methylthiophenyl)-imidazo(4,5-c)pyridin
2-(2-Ethoxy-4-methylthiophenyl)-imidazo(4,5-c)pyridin
2-(4-Ethoxy-2-methylthiophenyl)-imidazo(4,5-c)pyridin
2-(2-Allyloxy-4-methylthiophenyl)-imidazo(4,5-c)-pyridin
2-(4-Allyloxy-2-methylthiophenyl)-imidazo(4,5-c)pyridin
2-(2-Methylthio-4-propargyloxyphenyl)-imidazo(4,5-c)-pyridin
2-(4-Methylthio-2-propargyloxyphenyl)-imidazo(4,5-c)-pyridin
2-(2-Cyanmethoxy-4-methylthiophenyl)-imidazo(4,5-c)-pyridin
2-(4-Cyanmethoxy-2-methylthiophenyl)-imidazo(4,5-c)-pyridin
2-(2,4-Bis-methylthiophenyl)-imidazo(4,5-c)pyridin
2-(4-Ethylthio-2-methoxyphenyl)-imidazo(4,5-c)pyridin, Hydrochlorid, F. 262°
2-(2-Methoxy-4-propylthiophenyl)-imidazo(4,5-c)-pyridin
2-(4-Allylthio-2-methoxyphenyl)-imidazo(4,5-c)pyridin
2-(2-Methoxy-4-propargylthiophenyl)-imidazo(4,5-c)-pyridin
2-(4-Cyanmethylthio-2-methoxyphenyl)-imidazo-(4,5-c)pyridin
2-(4-Methoxycarbonylmethylthio-2-methoxyphenyl)-imidazo(4,5-c)pyridin
2-(4-Ethoxycarbonylmethylthic-2-methoxyphenyl)-imidazo(4,5-c)pyridin
2-(2-Hydroxy-4-methylsulfinylphenyl)-imidazo-(4,5-c)pyridin
2-(4-Hydroxy-2-methylsulfinylphenyl)-imidazo-(4,5-c)pyridin
2-(2-Methoxy-4-methylsulfinylphenyl)-imidazo-(4,5-c)pyridin, Fumarat, F. 215°
2-(4-Methoxy-2-methylsulfinylphenyl)-imidazo-(4,5-c)pyridin
2-(2-Ethoxy-4-methylsulfinylphenyl)-imidazo-(4,5-c)pyridin
2-(4-Ethoxy-2-methylsulfinylphenyl)-imidazo-(4,5-c)pyridin
2-(2-Allyloxy-4-methylsulfinylphenyl)-imidazo-(4,5-c)pyridin
2-(4-Allyloxy-2-methylsulfinylphenyl)-imidazo-(4,5-c)pyridin
2-(2-Methylsulfinyl-4-propargyloxyphenyl)-imidazo-(4,5-c)pyridin
2-(4-Methylsulfinyl-2-propargyloxyphenyl)-imidazo-(4,5-c)pyridin
2-(2-Cyanmethoxy-4-methylsulfinylphenyl)-imidazo-(4,5-c)pyridin
2-(4-Cyanmethoxy-2-methylsulfinylphenyl)-imidazo-(4,5-c)pyridin
2-(2,4-Bis-methylsulfinylphenyl)-imidazo(4,5-c)-pyridin
2-(4-Ethylsulfinyl-2-methoxyphenyl)-imidazo(4,5-c)-pyridin
2-(2-Methoxy-4-propylsulfinylphenyl)-imidazo(4,5-c)-pyridin
2-(4-Allylsulfinyl-2-methoxyphenyl)-imidazo(4,5-c)-pyridin
2-(2-Methoxy-4-propargylsulfinylphenyl)-imidazo-(4,5-c)pyridin
2-(4-Cyanmethylsulfinyl-2-methoxyphenyl)-imidazo-(4,5-c)pyridin
2-(4-Methoxycarbonylmethylsulfinyl-2-methoxyphenyl)-imidazo(4,5-c)pyridin
2 (4-Ethoxycarbonylmethylsulfinyl-2-methoxyphenyl)-imidazo(4,5-c)pyridin.

**Beispiel 2**

Eine Lösung von 176 mg p-Propargyloxybenzoesäure in 1 ml Pyridin wird mit einer Lösung von 109 mg IIa in 1 ml Pyridin versetzt, wobei das entsprechende Salz ausfällt. Unter Rühren tropft man bei 0° 0,19 ml $SOCl_2$ hinzu, rührt 1 Stunde bei 0° und 1 Stunde bei 70°, dampft ein und nimmt mit verdünnter Salzsäure auf. Das erhaltene Hydrochlorid wird in-Natriumcarbonatlösung eingetragen, das ausgefallene 2-(p-Propargyloxyphenyl)-imidazo(4,5-b)pyridin wird abfiltriert. F. 218°.

**Beispiel 3**

Man kocht 2,06 g 2-Methoxy-4-propargyloxy-benzoesäure mit 7 ml Benzol und 4 ml Thionylchlorid 1 Stunde, dampft ein und löst in 5 ml Benzol. Die Lösung des Säurechlorids wird zu einem Gemisch aus 1,09 g IIa 7 ml Pyridin und 5 ml Triethylamin getropft. Man rührt noch 2 Stunden bei 20°, versetzt mit Wasser, neutralisiert mit Salzsäure und arbeitet wie üblich auf. Das erhaltene rohe 2-Amino-3-(2-methoxy-4-propargyloxybenzoylamino)-pyridin wird in das Hydrochlorid überführt und dieses (200 mg) in 2 ml Pyridin gelöst. Unter Rühren bei 20° tropft man 0,2 ml $POCl_3$ hinzu, gießt nach 2 Stunden in Wasser, arbeitet wie üblich auf und erhält "M".

**Beispiel 4**

Ein Gemisch aus 10,9 g IIa 22 g 2-Methoxy-4-propargyl-oxy-benzoesäuremethylester und 300 ml $POC1_3$ wird 2 Stunden auf 120° erhitzt, eingedampft und der Rückstand mit 2n Salzsäure behandelt. Man erhält "M".

**Beispiel 5**

Ein Gemisch aus 1,09 g IIa, 1,87 g 2-Methoxy-4-propargyl-oxy-benzonitril und 3 g p-Toluolsulfonsäuremonohydrat wird 3,5 Stunden auf 160° erhitzt. Nach dem Abkühlen arbeitet man wie üblich auf und erhält "M".

**Beispiel 6**

Man erhitzt ein Gemisch aus 4,33 g S-Methyl-2-methoxy-4-propargyloxy-thiobenzoesäure-morpholid-jodid (erhältlich durch Kochen von 2-Methoxy-4-propargyloxy-benzaldehyd mit Schwefel in Morpholin und nachfolgende Umsetzung mit $CH_3J$ in Aceton), 1,09 g IIa und 35 ml Ethylenglykol 40 Minuten auf 130°, gießt in Eiswasser, filtriert und erhält "M".

**Beispiel 7**

Man erhitzt 1,09g IIa und 3 g 2-Methoxy-4-propargyloxy-benzoesäure-anhydrid 5 Stunden auf 180°, kühlt ab, arbeitet wie üblich auf und erhält "M".

**Beispiel 8**

Man mischt 2,78 g 2-Methoxy-4-propargyloxy-benzoesäure-morpholid mit 1,09 g IIa, tropft unter Rühren 5 ml $POC1_3$ hinzu, kocht 3 Stunden und dampft ein. Nach üblicher Aufarbeitung erhält man "M".

**Beispiel 9**

Man erhitzt 1,09 g IIa,3,11 g 2-Methoxy-4-propargyloxy-benzoesäure-morpholid-imidchlorid, 6 ml Triethylamin und 5 ml Diethylenglykol-dimethylether 30 Minuten auf 120°. Nach dem Erkalten arbeitet man wie üblich auf und erhält "M".

**Beispiel 10**

Man rührt 10,9 g IIa,19 g 2-Methoxy-4-propargyloxy-benzaldehyd (erhältlich aus 2,4-Dihydroxybenzaldehyd über 2-Hydroxy-4-propargyloxy-benzaldehyd) und 10 g Schwefel in 200 ml Mesitylen 10 Stunden bei 180°, dampft ein, extrahiert mit Methanol, filtriert und konzentriert die Lösung auf 350 ml. Durch Zugabe von etherischer Salzsäure fällt "M" aus.

**Beispiel 11**

Man löst 10 9 g IIa und 19 g 2-Methoxy-4-propargyloxy-benzaldehyd in 100 ml Dimethylacetamid, rührt nach Zugabe von 19 g Natriumdisulfit zwei Stunden bei 14o°, arbeitet wie üblich auf und erhält "M".

Analog Beispiel 10 oder 11 erhält man aus IIa oder 3,4-Diaminopyridin mit den entsprechenden Aldehyden (wie z.B. p-Propargyloxybenzaldehyd, F. 80°; m-Propargyloxybenzaldehyd, Öl; 2-Methoxy-4-ethoxycarbonylmethoxy-benzaldehyd, F.88°) die in Beispiel 1 angegebenen Verbindungen.

**Beispiel 12**

Man löst 24,1 g 2-(4-Hydroxy-2-methoxyphenyl)-imidazo-(4,5-b)pyridin [Hydrochlorid, F. 255°; erhältlich durch Kondensation von IIa mit 4-Benzyloxy-2-methoxy-benzoe-säure (F. 130°) zu 2-(4-Benzyloxy-2-methoxyphenyl)-imidazo (4,5-b)pyridin (Hydrochlorid, F. 244°) und anschließende Hydrogenolyse] in der berechneten Menge 2n Natronlauge, dampft ein und entfernt Reste von Wasser durch zweimalige Zugabe von Toluol und Eindampfen. Das erhaltene Na-Salz wird in 300 ml Dimethylformamid aufgenommen, mit 8 ml propargylchlorid versetzt und 16 Stunden bei 20° gerührt. Man arbeitet mit Wasser und Ethylacetat wie üblich auf und erhält "M".

Analog erhält man aus den entsprechenden Hydroxyverbindungen durch Veretherung die in Beispiel 1 angegebenen Ether.

**Beispiel 13**

Ein Gemisch von 10 g 2-(4-Acetoxy-2-methoxyphenyl)-imidazo (4, 5-c)pyridin, 100 ml Methanol und 100 ml 2n wässerige NaOH-Lösung wird 16 Stunden bei 20° stehengelassen. Nach üblicher Aufarbeitung erhält man 2-(4-Hydroxy-2-methoxyphenyl)-imidazo(4,5-c)pyridin; Fumarat, F. 255°.

Analog erhält man durch alkalische Hydrolyse der entsprechenden Acetoxyverbindungen die in Beispiel 1 genannten Hydroxyverbindungen.

**Beispiel 14**

Man hydriert eine Lösung von 10 g 2-(4-Benzyloxy-2-methoxyphenyl)-imidazo(4,5-c)pyridin-hydrochlorid (F. 241o; erhältlich durch Kondensation von IIb mit 4-Benzyloxy-2-methoxy-benzoesäure) in 150 ml Methanol an 5 g 5%igem Pd-C bei 20° und 1 bar bis zum Ende der Wasserstoffaufnahme, filtriert, arbeitet wie üblich auf und erhält 2-(4-Hydroxy-2-methoxyphenyl)-imidazo(4,5-c)pyridin; Fumarat, F. 255°.

Analog erhält man durch Hydrogenolyse der entsprechenden Benzylether bzw. Benzylthioether die in Beispiel 1 genannten Hydroxy- bzw. Mercaptoverbindungen.

**Beispiel 15**

Analog Beispiel 12 erhält man durch Umwandlung von 2-(4-Mercapto-2-methoxyphenyl)-imidazo(4,5-c)pyridin in das Na-Salz und nachfolgende Reaktion mit Methyljodid das 2-(2-Methoxy-4-methylthiophényl)-imidazo-(4,5-c)pyridin, Hydrochlorid, F. 253°.

Analog erhält man aus den entsprechenden Mercaptoverbindungen durch Thio-veretherung die in Beispiel 1 beschriebenen Thioether.

**Beispiel 16**

Zu einer siedenden Lösung von 2,71 g 2-(2-Methoxy-4-methylthio-phenyl)-imidazo(4,5-c)pyridin in 50 ml Ethanol gibt man 10 ml 30%iges $H_2O_2$ und kocht anschließend 3 Stunden. Nach dem Abkühlen und üblicher Aufarbeitung erhält man 2-(2-Methoxy-4-methylsulfinylphenyl)-imidazo(4,5-c)pyridin; Fumarat, F. 215°.

Analog erhält man durch Oxydation der entsprechenden Thioether die in Beispiel 1 beschriebenen Sulfinylverbindungen.

Die nachstehenden Beispiele betreffen pharmazeutische Zubereitungen,die Verbindungen der Formel I oder ihre Säureadditionssalze enthalten:

**Beispiel A: Tabletten**

Ein Gemisch von 1 kg "M", 4 kg Lactose, 1,2 kg Kartoffelstärke, 0,2 kg Talk und 0,1 kg Magnesiumstearat wird in üblicher Weise zu Tabletten gepreßt, derart, daß jede Tablette 100 mg Wirkstoff enthält.

**Beispiel B: Dragees**

Analog Beispiel A werden Tabletten gepreßt, die anschließend in üblicher Weise mit einem Überzug aus Saccharose, Kartoffelstärke, Talk, Tragant und Farbstoff überzogen werden.

**Beispiel C: Kapseln**

10 kg "M" werden in üblicher Weise in Hartgelatinekapseln gefüllt, so daß jede Kapsel 50 mg Wirkstoff enthält.

**Beispiel D: Ampullen**

Eine Lösung von 1 kg "M" in 100 1 zweifach destilliertem Wasser wird steril filtriert, in Ampullen abgefüllt, unter sterilen Bedingungen lyophilisiert und steril verschlossen. Jede Ampulle enthält 50 mg Wirkstoff.

Analog sind Tabletten, Dragees, Kapseln und Ampullen, erhältlich, die einen oder mehrere der übrigen Wirkstoffen der Formel I und/oder ihre physiologisch unbedenklichen Säureadditionssalze enthalten.

**Patentansprüche**

für die Vertragsstaaten BE, CH, DE, FR, GB, IT, LI, NL, SE

1. 2-Aryl-imidazopyridine der allgemeinen Formel I

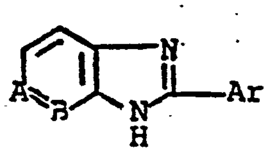

I

worin

-A=B- (a) -CH=N- oder (b) -N=CH-,

Ar einen Phenylrest, der im Fall (a) durch eine oder zwei Alkinyloxy-, Cyanmethoxy-, und/oder Alkyloxycarbonylmethoxygruppen substituiert ist und durch eine oder zwei zusätzliche Hydroxy-, Alkyloxy-, Alkenyloxy- und/oder Alkinyloxygruppen substituiert sein kann, oder der im Fall (b) durch eine bis drei Hydroxy-, Mercapto- und/oder -Z-R-Gruppen substituiert ist,

Z -O-, -S- oder -SO- und

R Alkyl, Alkenyl, Alkinyl, Hydroxyalkyl, Cyanmethyl oder Alkyloxycarbonylmethyl bedeuten,

wobei die Alkyl-, Alkenyl-, Alkinyl- und Hydroxyalkylgruppen jeweils bis zu 5 C-Atome besitzen, worin jedoch im Fall (b) der Phenylrest nur dann durch Hydroxy- oder Methoxygruppen substituiert ist, wenn er gleichzeitig noch mindestens einen anderen jeweils davon verschiedenen Substituenten trägt,

sowie ihre physiologisch unbedenklichen Salze.

2. 2-(2-Methoxy-4-propargyloxyphenyl)-imidazo(4,5-b)-pyridin.

3. 2-(4-Cyanmethoxy-2-methoxyphenyl)-imidazo(4,5-b)-pyridin.

4. 2-(4-Allyloxy-2-methoxyphenyl)-imidazo(4,5-c)-pyridin.

5. 2-(2-Methoxy-4-propargyloxyphenyl)-imidazo(4,5-c)-pyridin.

6. 2-(2-Methoxy-4-methylthiophenyl)-imidazo(4,5)pyridin.

7. 2-(Methoxy-4-methylsulfinylphenyl)-imidazo(4,5)pyridin.

8. Verfahren zur Herstellung von 2-Arylimidazopyridinen der allgemeinen Formel I gemäß Anspruch 1 sowie ihrer physiologisch unbedenklichen Salze,
dadurch gekennzeichnet, daß man ein Diaminopyridin der allgemeinen Formel II

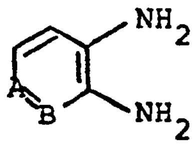

II

worin die Gruppe
-A=B- die in Anspruch 1 angegebene Bedeutung hat mit einer Benzoesäure der allgemeinen Formel III
HOOC-Ar III
worin
Ar die im Anspruch 1 angegebene Bedeutung hat
oder mit einem ihrer reaktionsfähigen Derivate
oder mit einem Aldehyd der allgemeinen Formel IV
OCH-Ar IV
worin
Ar die im Anspruch 1 angegebene Bedeutung hat
in Gegenwart eines Oxydationsmittels umsetzt

oder daß man eine Verbindung, die der Formel I entspricht, aber anstelle einer oder mehrerer Ethergruppen eine oder mehrere freie Hydroxygruppen enthält, mit einem Veretherungsmittel behandelt

oder daß man eine Verbindung, die der Formel I entspricht, aber anstelle einer oder mehrerer freier Hydroxy- und/oder Mercaptogruppen eine oder mehrere geschützte Hydroxy- und/oder Mercaptogruppen enthält, mit einem solvolysierenden oder hydrogenolysierenden Mittel behandelt

und daß man gegebenenfalls in dem erhaltenen Produkt Hydroxygruppen verethert und/oder Mercaptogruppen in Thioethergruppen überführt und/oder Thioethergruppen zu Sulfinylgruppen oxydiert

und/oder eine erhaltene Verbindung durch Behandeln mit einer Säure bzw. Base in eines ihrer physiologisch unbedenklichen Salze umwandelt.

9. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfestoff und gegebenenfalls in Kombination mit einem oder mehreren weiteren Wirkstoff(en) in eine geeignete Dosierungsform bringt.

10. Pharmazeutische Zubereitung, gekennzeichnet durch einen Gehalt an mindestens einer Verbindung der Formel I gemäß Anspruch 1 und/oder einem ihrer physiologisch unbedenklichen Salze.

11. Verbindungen der allgemeinen Formel I gemäß Anspruch 1 zur Bekämpfung von Krankheiten.

**Patentansprüche**

für den Vertragsstaat AT

1. Verfahren zur Herstellung von 2-Arylimidazopyriden der allgemeinen Formel I

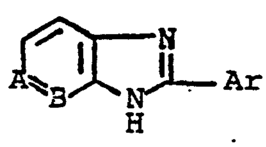

I

worin
-A=B- (a) -CH=N- oder (b) -N=CH-,
Ar einen Phenylrest, der

im Fall (a) durch eine oder zwei Alkinyloxy-, Cyanmethoxy- und/oder Alkyloxycarbonyl-, methoxygruppen substituiert ist und durch eine oder zwei zusätzliche Hydroxy-, Alkyloxy-, Alkenyloxy- und/oder Alkinyloxygruppen substituiert sein kann, oder der im Fall (b) durch eine bis drei Hydroxy-, Mercapto- und/oder -Z-R-Gruppen substituiert ist,

Z -O-, -S- oder -SO- und

R Alkyl, Alkenyl, Alkinyl, Hydroxyalkyl, Cyanmethyl oder Alkyloxycarbonylmethyl bedeuten,

wobei die Alkyl-, Alkenyl, Alkinyl, Hydroxyalkylgruppen jeweils bis zu 5 C-Atomen besitzen, worin jedoch im Fall (b) der Phenylrest nur dann durch Hydroxy- Methoxygruppen substituiert ist, wenn er gleichzeitig noch mindesten einen anderen jeweils davon verschiedenen Substituenten trägt,

sowie ihrer physiologisch unbedenklichen Salze, dadurch gekennzeichnet, daß man ein Diaminpyridin der allgemeinen Formel II

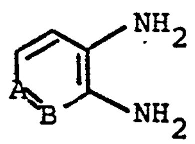

II

worin die Gruppe

-A=B- die oben angegebene Bedeutung hat mit einer Benzosäure der allgemeinen Formel III

HOOC-Ar III

worin

Ar die oben angegebene Bedeutung hat

oder mit einem ihrer reaktionsfähigen Derivate

oder mit einem Aldehyd der allgemeinen Formel IV

OCH-Ar IV

worin

Ar die oben angegebene Bedeutung hat in Gegenwart eines Oxydationsmittels

umsetzt·

oder daß man eine Verbindung, die der Formel I entspricht, aber anstelle einer oder mehrerer Ethergruppen eine oder mehrere freie Hydroxygruppen enthält, mit einem Veretherungsmittel behandelt oder daß man eine Verbindung, die der Formel I entspricht, aber anstelle einer oder mehrerer freier Hydroxy- und/oder Mercaptgruppen eine oder mehrere geschützte Hydroxy- und/oder Mercaptogruppen enthält, mit einem solvolysierenden oder hydrogenolysierenden Mittel behandelt

und daß man gegebenenfalls in dem erhaltenen Produkt Hydroxygruppen verethert und/oder Mercaptogruppen in Thioethergruppen überführt und/oder Thioethergruppen zu Sulfinylgruppen oxydiert

und/oder eine erhaltene Verbindung durch Behandeln mit einer Säure bzw. Base in eines ihrer physiologisch unbedenklichen Salze umwandelt.

2. Verfahren zur Herstellung von 2-Arylimidazo(4,5-b)-pyridinen der allgemeinen Formel I

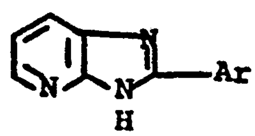

I

worin

Ar einen Phenylrest bedeutet, der durch eine oder zwei Alkinyloxy-, Cyanmethoxy- und/oder Alkyloxycarbonylmethoxygruppen substituiert ist und durch eine oder zwei zusätzliche Hydroxy-, Alkyloxy-, Alkenyloxy- und/oder Alkinyloxygruppen substituiert sein kann,

wobei die Alkyl-, Alkenyl- und Alkinylgruppen jeweils bis zu 5 C-Atome besitzen,

sowie ihrer physiologisch unbedenklichen Salze, dadurch gekennzeichnet, daß man 2,3-Diaminopyridin (II) mit einer Benzoesäure der allgemeinen Formel III

HOOC-Ar III

worin

Ar die oben angegebene Bedeutung hat

oder mit einem ihrer reaktionsfähigen Derivate

oder mit einem Aldehyd der allgemeinen Formel

OCH-Ar IV

worin

Ar die oben angegebene Bedeutung hat

in Gegenwart eines Oxydationsmittels

umsetzt

oder daß man eine Verbindung, die der Formel I entspricht, aber an Stelle einer oder mehrerer Ethergruppen eine oder mehrere freie Hydroxygruppen enthält, mit einem Veretherungsmittel behandelt

und daß man gegebenenfalls in dem erhaltenen Produkt Hydroxygruppen verethert

und/oder eine erhaltene Verbindung durch Behandeln mit einer Säure bzw. Base in eines ihrer physiologisch unbedenklichen Salze umwandelt.

3. Verfahren zur Herstellung von 2-Arylimidazo(4,5-c)-pyridinen der allgemeinen Formel I

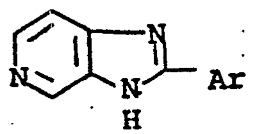

I

worin

Ar einen Phenylrest, der durch eine bis drei Hydroxy-, Mercapto- und/oder -Z-R-Gruppen substituiert ist,

Z -O-, -S- oder -SO- und

R Alkyl, Alkenyl, Alkinyl, Hydroxyalkyl, Cyanmethyl oder Alkyloxycarbonylmethyl bedeuten,

wobei die Alkyl-, Alkenyl-, Alkinyl- und Hydroxyalkylgruppen jeweils bis zu 5 C-Atome besitzen, worin jedoch der Phenylrest nur dann durch Hydroxy- oder Methoxygruppen substituiert ist, wenn er gleichzeitig noch mindestens einen anderen jeweils davon verschiedenen Substituenten trägt, sowie ihrer physiologisch unbedenklichen Salze, dadurch gekennzeichhet, daß man 3,4-Diaminopyridin (II) mit einer Benzoesäure der allgemeinen Formel III

HOOC-Ar III

worin

Ar die oben angegebene Bedeutung hat

oder mit einem ihrer reaktionsfähigen Derivate

oder mit einem Aldehyd der allgemeinen Formel IV

OCH-Ar IV

worin

Ar die oben angegebene Bedeutung hat in Gegenwart eines Oxydationsmittels umsetzt

oder daß man eine Verbindung, die der Formel I entspricht, aber anstelle einer oder mehrerer freier Hydroxy- und/oder Mercaptogruppen eine oder mehrere geschützte Hydroxy- und/oder Mercaptogruppen enthält, mit einem solvolysierenden oder hydrogenolysierenden Mittel behandelt

und daß man gegebenenfalls in dem erhaltenen Produkt Hydroxygruppen verethert und/oder Mercaptogruppen in Thioethergruppen überführt und/oder Thioethergruppen zu Sulfinylgruppen oxydiert

und/oder eine erhaltene Verbindung durch Bahandeln mit einer Säure bzw. Base in eines ihrer physiologisch unbedenklichen Salze umwandelt.

**Claims**

for the Contracting states BE, CH, DE, FR, GB, IT, LI, NL, SE

1. 2-Arylimidazopyridines of the general formula I

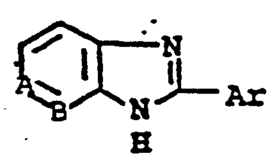

I

wherein

-A=B- is (a) -CH=N- or (b) -N=CH- Ar is a phenyl radical, which, in case (a), is substituted by one or two alkinyloxy cyanomethoxy and/or alkyloxycarbonylmethoxy groups and can be substituted by one or two additional hydroxyl alkyloxy alkenyloxy and/or alkinyloxy groups or which in case (b) is substituted by one to three hydroxyl and/or -Z-R groups

Z is -O- -S- or -SO- and

R is alkyl alkenyl alkinyl hydroxyalkyl, cyanomethyl or alkyloxycarbonylmethyl, the alkyl, alkenyl, alkiny and hydroxyalkyl groups each having up to 5 C atoms, but wherein in case (b), the phenyl radical is only substituted by hydroxyl or methoxy groups if it has at the same time at least one other substituent differing from these,

and their physiologically acceptable salts.

2. 2-(2-Methoxy-4-propargyloxyphenyl)imidazo(4,5-b)-pyridine.
3. 2-(4-Cyanomethoxy-2-methoxyphenyl)imidazo(4,5-b-pyridine.
4. 2-(4-Allyloxy-2-methoxyphenyl)imidazo(4,5-c)-pyridine.
5. 2-(2-Methoxy-4-propargyloxyphenyl)imidazo(4,5-c)-pyridine.
6. 2-(2-Methoxy-4-methylthiophenyl)-imidazo(4,5-c)-pyridine.
7. 2-(2-Methoxy-4-methylsulfinylphenyl)-imidazo(4,5-c)-pyridine.
8. Process for the preparation of 2-arylimidazo-pyridines of the general formula 1 according to claim 1 and their physiologically acceptable salts, characterised in that a diaminopyridine of the general formula II

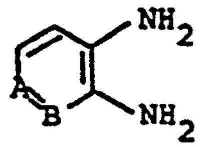

II

wherein
the group -A=B- has the the meaning mentioned in claims 1, is reacted with a bezoic acid of the general formla III
HOOC-Ar III
wherein
Ar has the meaning mentioned in claim 1,
in the presence of an oxidising agent
or in that a compound which corresponds to the formula I but which contains one or more free hydroxyl groups instead of one or more ether groups is treated withan etherifying agent or in that a compound which corresponds to the formula I but which contains one or more protected hydroxyl and/or mercapto groups instead of one or more free hydroxyl and/or mercapto groups, is treated with a solvolysing or hydrogenolysing agent and in that if desired, hydroxyl groups in the product obtained are etherified and/or mercapto groups are converted into thioether groups and/or thioether groups are oxidised to sulfinyl groups
and/or a compound obtained is converted into one of its physiologically acceptable salts by treatment with an acid or base.
9. Process for the preparation of pharmaceutical formulations,characterised in that a compound of the formula I according to claim 1 and/or one of its physiologically acceptable salts is converted into a form suitable for administration together with at least one solid, liquid or semiliquid vehicle or auxiliary and optionally in combination with one or more other active ingredients.
10. Pharmaceutical formulation characterised by a content of at least one compound of the formula I according to claim 1 and/or one of its physiologically acceptable salts.
11. Compounds of the general formula I according to claim 1 for combating diseases.

**Claims**

for the Contracting state: AT
1. Process for the preparation of 2-arylimidazo-pyridines of the general formula I

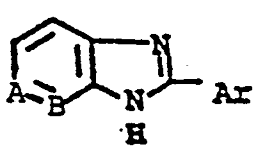

I

wherein
-A=B- is (a) -CH=N- or (b) -N=CH-,
Ar is a phenyl radical, which, in case (a), is substitutet by one or two alkinyloxy, cyanomethoxy and/or alkyloxycarbonylmethoxy groups and can be substituted by one or two additional hydroxyl, alkyloxy, alkenyloxy, and/or alkinyloxy groups, or which, in case (b), is substituted by one to three hydroxyl, mercapto and/or -Z-R groups
Z is -O-, -S- or -SO- and
R is alkyl, alkenyl, alkinyl, hydroxyalkyl, cyanomethyl, carboxymethyl or alkyloxycarbonylmethyl
the alkyl, alkenyl, alkinyl and hydroxyalkyl groups each having up to 5 C atoms, but wherein in case (b) the phenyl radical is only substituted by hydroxyl or methoxy groups if it has at the same time at least one other

substituent differing from these

and their physiologically acceptable salts, characterised in that a diamimopyridine of the general formula II

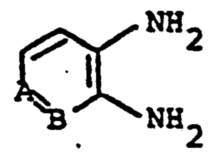

II

wherein

the group -A=B- has the above-mentioned meaning is reacted with a benzoic acid of the general formula I

HOOC-Ar III

wherein

Ar has the above-mentioned meaning

or with one of its reactive derivates or with an aldehyde of the general formula IV

OCH-Ar IV

wherein

Ar has the above-mentioned meaning,

in the presence of am oxidising agent

or in that a compound which corresponds to the formula I but which contains one or more free hydroxyl groups instead of one or more ether groups, is treated with an etherifying agent or in that a compound which corresponds to the formula I but which contains one or more protected hydroxyl and/or mercapto groups instead of one or more free hydroxyl and/or mercapto groups, is treated with a solvolysing or hydrogenolysing agent and in that if desired, hydroxyl groups in the product obtained are etherified and/or mercapto groups are converted into thioether groups and/or thioether groups are oxidised to sulfinyl groups

and/or a compound obtained is converted into one of its physiologically acceptable salts by treatment with an acid or base.

2. Process for the preparation of 2-arylimidazo(4,5-b)-pyridines of the general formula I

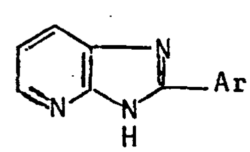

I

wherein

Ar is a phenyl radical, which is substitued by and or two alkinyloxy, cyanomethoxy and/or alkyloxycarbonylmethoxy groups and can be substituted by one or two additional hydroxyl, alkyloxy, alkenyloxy and/or alkinyloxy groups,

the alkyl, alkanyl and alkinyl groups each having up to 5 C atoms,

and their physiologically acceptable salts,

characterised in that 2,3-diaminopyridine (II) is reacted with a benzoic acid of the general formula III

HOOC-Ar III

wherein

Ar has the above-mentioned meaning

or with one of its reactive derivatives or with an aldehyde of the general formula IV

OCH-Ar IV

wherein

Ar has the above-mentioned meaning, in the presence of an oxidising agent

or in that a compound which corresponds to the formula I but which contains one or more free hydroxyl groups instead of one or more ether groups, is treated with an etherifying agent, and in that, if desired, hydroxyl groups in the product obtained are etherified

and/or a compound obtained is converted into one of its physiologically acceptable salts by treatment with an acid or base.

3. Process for the preparation of 2-arylimidazo(4,5-c)-pyridines of the general formula I

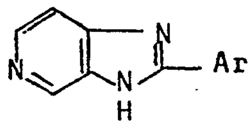

I

wherein
Ar is a phenyl radical, which is substituted by one to three hydroxyl, mercapto and/or-Z-R groups,
Z is -O-, -S- or -SO- and
R is alkyl, alkenyl, alkinyl, hydroxyalkyl, cyanomethyl or alkyloxycarbonylmethyl,
the alkyl, alkenyl, alkinyl and hydroxyalkyl groups each having up to 5 C atoms but wherein the phenyl radical is only substituted by hydroxyl or methoxy groups if it has at the same time at least one other substituent differing from these
and their physiologically acceptable salts,
characterised in that 3,4-diaminopyridine (II) is reacted with a benzoic acid of the general formula III
HOOC-Ar III
wherein
Ar has the above-mentioned meaning
or with one of its reactive derivatives or with an aldehyde of the general formula IV
OCH-Ar IV
wherein
Ar has the above-mentioned meaning, in the presence of an oxidising agent or in that a compound which corresponds to the formula I but which contains one or more protected hydroxyl and/or mercapto groups instead of one or more free hydroxyl and/or mercapto groups is treated with a solvolysing or hydrogenolysing agent and in that if desired, hydroxyl groups in the product obtained are etherified and/or mercapto groups are converted into thioether groups and/or thioether groups are oxidised to sulfinyl groups
and/or a compound obtained is converted into one of its physiologically acceptable salts by treatment with an acid or base.

**Revendications**

1. 2-aryl-imidazopyridines de formule générale I:

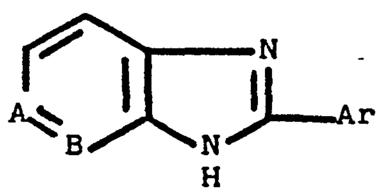

I

dans laquelle
-A=B- représente (a) -CH=N- ou (b) -N=CH-,
Ar représente un groupe phényle qui, dans le cas (a), est substitué par un ou deux groupes alcynyloxy, cyanométhoxy et/ou alkyloxycarbonylmethoxy et que peut être substitue par un ou deux groupes hydroxy, alkyloxy, alcényloxy et/ou alcynyloxy supplémentaires, ou qui, dans le cas (b), est substitué par un à trois groupes hydroxy, mercapto et/ou -Z-R-,
Z représente -O-, -S- ou -SO- et
R représente un groupe alkyle, un groupe alcényle, un groupe alcynyle, un groupe hydroxyalkyle, un groupe cyanométhyle ou un groupe alkyloxycarbonylméthyle,
les groupes alkyle, alcényle, alcynyle et hydroxyalkyle contenant chacun jusqu'à 5 atomes de carbone, cependant que, dans le cas (b), le groupe phényle n'est substitué que par des groupes hydroxy ou méthoxy lorsqu'il comporte simultanément encore au moins un autre substituant chaque fois différent, de même que leurs sels physiologiquement acceptables.

2. La 2-(2-méthoxy-4-propargyloxyphényl)-imidazo(4,5-b)pyridine.
3. La 2-(4-cyanométhoxy-2-méthoxyphényl)-imidazo(4,5-b)pyridine.
4. La 2-(4-allyloxy-2-méthoxyphényl)-imidazo(4,5-c)pyridine.
5. La 2-(2-méthoxy-4-propargyloxyphényl)-imidazo(4,5-c)pyridine.
6. La 2-(2-méthoxy-4-méthylthiophényl)-imidazo(4,5-c)pyridine.
7. La 2-(2-méthoxy-4-méthylsulfinylphé-nyl)-imidazo(4,5-c)pyridine.
8. Procédé de préparation de 2-arylimidazo-pyridines de formule générale I selon la revendication 1,

ainsi que de leurs sels physiologiquement acceptables, caractérisé en ce qu'on fait réagir une diaminopyridine de formule générale II:

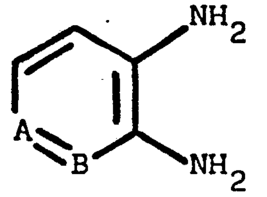

II

dans laquelle
le groupe -A=B- a la signification indiquée dans la revendication 1,
avec un acide benzoïque de formule générale III:
HOOC-Ar
dans laquelle
Ar a la signification indiquée dans la revendication 1, ou avec un de ses dérivés réactifs ou encore avec un aldéhyde de formule générale IV:
OCH-Ar IV
dans laquelle
Ar a la signification indiquée dans la revendication 1, en présence d'un agent d'oxydation,
ou on traite un composé qui répond à la formule I mais qui, au lieu d'un ou plusieurs groupes éther, contient un ou plusieurs groupes hydroxy libres, avec un agent d'éthérification ou on traite un composé qui répond à la formule I mais qui, au lieu d'un ou plusieurs groupes hydroxy et/ou mercapto libres, contient un ou plusieurs groupes hydroxy et/ou mercapto protégés, avec un agent solvolysant ou hydrogénolysant
et on éthérifie éventuellement les groupes hydroxy dans le produit obtenu et/ou on transforme les groupes mercapto en groupes thioéther et/ou on oxyde les groupes thioéther en groupes sulfinyle et/ou on transforme un composé obtenu en un de ses sels physiologiquement acceptables par traitement avec un acide ou une base.

9. Procédé en vue d'obtenir des préparations pharmaceutiques, caractérisé en ce qu'on met, sous une forme de dosage appropriée, un composé de formule I selon la revendication 1 et/ou un de ses sels physiologiquement acceptables avec au moins une substance auxiliaire ou une substance support solide, liquide ou semi-liquide et éventuellement en combinaison avec une ou plusieurs autres substances actives.

10. Préparation pharmaceutique, caractérisée en ce qu'elle contient au moins un composé de formule I selon la revendication 1 et/ou un de ses sels physiologiquement acceptables.

11. Composés de formule générale I selon la revendication 1, en vue de combattre les maladies.

**Revendications**

pour l'Etat contractant: AT

1. Procédé de préparation de 2-arylimidazo-pyridines de formule générale I:

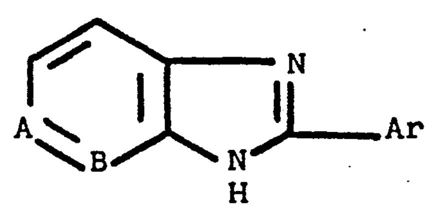

I

dans laquelle
-A=B- représente (a) -CH=N- ou (b) -N=CH-,
Ar représente un groupe phényle qui, dans le cas (a), est substitué par un ou deux groupes alcynyloxy, cyanométhoxy et/ou alkyloxycarbonylméthoxy et qui peut être substitué par un ou deux groupes hydroxy, alkyloxy, alcényloxy et/ou alcynyloxy supplémentaires ou encore qui, dans le cas (b), est substitué par un à

trois groupes hydroxy, mercapto et/ou -Z-R-,

Z représente -O-, -S- ou -SO- et

R représente un groupe alkyle, un groupe alcényle, un groupe alcynyle, un groupe hydroxyalkyle, un groupe cyanométhyle ou un groupe alkyloxycarbonylméthyle,

les groupes alkyle, alcényle, alcynyle et hydroxyalkyle contenant chacun jusqu'à 5 atomes de carbone cependant que, dans le cas (b), le groupe phényle n'est substitué que par des groupes hydroxy ou méthoxy lorsque simultanément il comporte encore au moins un autre substituant chaque fois différent, ainsi que de leurs sels physiologiquement acceptables, caractérisé en ce qu'on fait réagir une diaminopyridine de formule générale II:

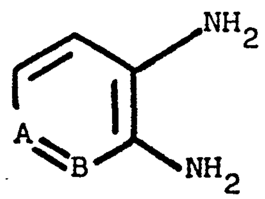

II

dans laquelle

le groupe -A=B- a la signification indiquée ci-dessus avec un acide benzoïque de formule générale III

HOOC-Ar III

dans laquelle

Ar a la signification indiquée ci-dessus, ou avec un de ses dérivés réactifs ou encore avec un aldéhyde de formule générale IV:

OCH-Ar IV

dans laquelle

Ar a la signification indiquée ci-dessus, en présence d'un agent d'oxydation,

ou on traite un composé qui répond à la formule I mais qui, au lieu d'un ou plusieurs groupes éther, contient un ou plusieurs groupes hydroxy libres, avec un agent d'éthérification ou on traite un composé qui répond à la formule I mais qui, au lieu d'un ou plusieurs groupes hydroxy et/ou mercapto libres, contient un ou plusieurs groupes hydroxy et/ou mercapto protégés, avec un agent solvolysant ou hydrogénolysant,

et on éthérifie éventuellement des groupes hydroxy dans le produit obtenu et/ou on transforme des groupes mercapto en groupes thioéther et/ou on oxyde des groupes thioéther en groupes sulfinyle

et/ou on transforme un composé obtenu en un de ses sels physiologiquement acceptables par traitement avec un acide ou une base.

2. Procédé de préparation de 2-arylimidazo-(4,5-b)pyridines de formule générale I:

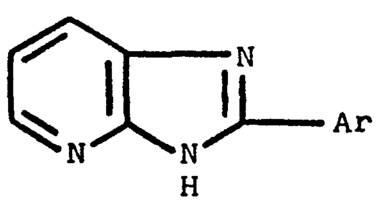

I

dans laquelle

Ar représente un groupe phényle qui est substitué par un ou deux groupes alcynyloxy, cyanométhoxy et/ou alkyloxycarbonylméthoxy et qui peut être oxy, alcényloxy et/ou alcynyloxy supplémentaires, les groupes alkyle, alcényle et alcynyle contenant chacun jusqu'à 5 atomes de carbone, ainsi que de leurs sels physiologiquement acceptables, caractérisé en ce qu'on fait réagir une 2,3-diamino-pyridine (II) avec un acide benzoïque de formule générale III:

HOOC-Ar III

dans laquelle

Ar a la signification indiquée ci-dessus, ou avec un de ses dérivés réactifs ou encore avec un aldéhyde de formule générale IV:

OCH-Ar IV

dans laquelle

Ar a la signification indiquée ci-dessus, en presence d'un agent d'oxydation,

ou on traite un composé qui répond à la formule I mais qui, au lieu d'un ou plusieurs groupes éther, contient un ou plusieurs groupes hydroxy libres, avec un agent d'éthérification,

et on éthérifie éventuellement des groupes hydroxy dans le produit obtenu et/ou on transforme un compose obtenu en un de ses sels physiologiquement acceptables par traitement avec un acide ou une base.

3. Procédé de préparation de 2-arylimidazo-(4,5-c)pyridines de formule générale I:

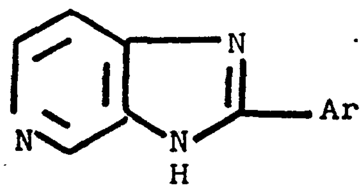

I

dans laquelle

Ar représente un groupe phényle qui est substitué par un à trois groupes hydroxy, mercapto et/ou -Z-R-,

Z représente -O-, -S- ou -SO- et

R représente un groupe alkyle, un groupe alcényle, un groupe alcynyle, un groupe hydroxyalkyle, un groupe cyanométhyle ou un groupe alkyloxycarbonylméthyle,

les groupes alkyle, alcényle, alcynyle et hydroxyalkyle contenant chacun jusqu'à 5 atomes de carbone, cependant que le groupe phényle n'est alors substitué que par des groupes hydroxy ou méthoxy lorsque simultanément il comporte encore au moins un autre substituant chaque fois différent, ainsi que de leurs sels physiologiquement acceptables, caractérisé en ce qu'on fait réagir une 3,4-diaminopyri-dine (II) avec un acide benzoïque de formule générale III:

HOOC-Ar III

dans laquelle

Ar a la signification indiquée ci-dessus, ou avec un de ses dérivés réactifs ou enoore avec un aldéhyde de formule générale IV:

OCH-Ar IV

dans laquelle

Ar a la signification indiquée ci-dessus, en présence d'un agent d'oxydation, on on traite un composé qui répond à la formule I mais qui, au lieu d'un ou plusieurs groupes hydroxy et/ou mercapto libres, contient un ou plusieurs groupes hydroxy et/ou mercapto protégés, avec un agent solvolysant ou hydrogénolysant, et on éthérifie éventuellement des groupes hydroxy dans le produit obtenu et/ou on transforme des groupes mercapto en groupes thioéther et/ou on oxyde des groupes thioéther en groupes sulfinyle et/ou on transforme un composé obtenu en un de ses sels physiologiquement acceptables par traitement avec un acide ou une base.